# EUROPEAN PATENT APPLICATION

(11) **EP 1 738 762 A1**
(43) Date of publication of application: **03.01.2007**
(21) Application number: 05727384.9
(22) Date of filing: 25.03.2005
(51) Int. Cl.: A61K 38/00, A61P 3/08, A61P 3/10, A61P 9/10, A61P 13/12, A61P 25/02, C07K 5/11

(54) **PREVENTIVE/REMEDY FOR DIABETIC COMPLICATIONS USING OLIGOPEPTIDE**

(30) Priority: 26.03.2004 JP 2004091767
(71) Applicant: Ajinomoto Co., Inc., Tokyo 104-8315 (JP)
(72) Inventor: KITAHARA, Yoshiro, c/o Ajinomoto Co., Inc.,, Kawasaki-shi, Kanagawa 2108681 (JP); TAKEUCHI, Masayoshi, 9330321 (JP)
(74) Representative: HOFFMANN EITLE
(86) International application number: PCT/JP2005/006432
(87) International publication number: WO 2005/092363

(57) **Abstract**

The present invention provides a novel agent for the prophylaxis or treatment of diabetic complications. Specifically, the present invention provides an agent for the prophylaxis or treatment of diabetic complications, which contains, as an active ingredient, an oligopeptide containing at least two amino acid residues having an amino group or a guadinino group in the side chain, and which has AGE2 production inhibitory action and AGE2 blood concentration reduction promoting action.

SEQUENCE LISTING

<110>  Ajinomoto Co., Inc.

<120>  An agent for prophylaxis and/or treatment of diabetic complications using an oligopeptide

<130>  09730

<150>  JP2004-091767
<151>  2004-03-26

<160>  18

<210>  1
<211>  4
<212>  PRT
<213>  Artificial Sequence

<220>
<223>  Peptide having inhibitory action against AGE2 production

<400>  1
Lys Lys Lys Lys
1

<210>  2
<211>  4
<212>  PRT
<213>  Artificial Sequence

<220>
<223>  Peptide having inhibitory action against AGE2 production

<220>
<221>  MISC_FEATURE
<222>  (2)..(2)
<223>  Xaa shows any amino acid.

<220>
<221>  MISC_FEATURE
<222>  (4)..(4)
<223>  Xaa shows any amino acid.

<400>  2
Lys Xaa Lys Xaa
1

<210>  3
<211>  4
<212>  PRT
<213>  Artificial Sequence

<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (4)..(4)
<223>  Xaa shows any amino acid.

<400>  3
Xaa Lys  Lys Xaa
1

<210>  4
<211>  4
<212>  PRT
<213>  Artificial Sequence

<220>
<223>  Peptide having inhibitory action against AGE2 production

<220>
<221>  MISC_FEATURE
<222>  (2)..(2)
<223>  Xaa shows any amino acid.

<220>
<221>  MISC_FEATURE
<222>  (4)..(4)
<223>  Xaa shows any amino acid.

<400>  4
Arg Xaa Arg Xaa
1


<210>  5
<211>  4
<212>  PRT
<213>  Artificial Sequence

<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (4)..(4)
<223>  Xaa shows any amino acid.

<400>  5
Xaa Arg Arg Xaa
1


<210>  6
<211>  4
<212>  PRT
<213>  Artificial Sequence

<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (4)..(4)
<223>  Xaa shows any amino acid.

<400>  6
Xaa Lys Arg Xaa
1


<210>  7
<211>  4
<212>  PRT
<213>  Artificial Sequence

<220>
<223>  Peptide having inhibitory action against AGE2 production

<220>
<221>  MISC_FEATURE
<222>  (2)..(2)

<223>  Xaa shows any amino acid.

<220>
<221>  MISC_FEATURE
<222>  (4)..(4)
<223>  Xaa shows any amino acid.

<400>  7
Lys Xaa Arg Xaa
1


<210>  8
<211>  4
<212>  PRT
<213>  Artificial Sequence
<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (3)..(3)
<223>  Xaa shows any amino acid.

<400>  8
Xaa Lys Xaa Arg
1


<210>  9
<211>  4
<212>  PRT
<213>  Artificial Sequence

<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (2)..(2)
<223>  Orn

<220>
<221>  MISC_FEATURE
<222>  (4)..(4)
<223>  Xaa shows any amino acid.

<400>  9
Xaa Xaa Lys Xaa
1


<210>  10
<211>  4
<212>  PRT
<213>  Artificial Sequence

<220>
<223>  Peptide having inhibitory action against AGE2 production

<220>
<221>  MISC_FEATURE
<222>  (1)..(1)
<223>  Orn

<220>

<221>  MISC_FEATURE
<222>  (2)..(2)
<223>  Xaa shows any amino acid.

<220>
<221>  MISC_FEATURE
<222>  (4)..(4)
<223>  Xaa shows any amino acid.

<400>  10
Xaa Xaa Lys Xaa
1


<210>  11
<211>  4
<212>  PRT
<213>  Artificial Sequence

<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (2)..(2)
<223>  Orn

<220>
<221>  MISC_FEATURE
<222>  (3)..(3)
<223>  Xaa shows any amino acid.

<400>  11
Xaa Xaa Xaa Lys
1


<210>  12
<211>  4
<212>  PRT
<213>  Artificial Sequence

<220>
<223>  Peptide having inhibitory action against AGE2 production

<220>
<221>  MISC_FEATURE
<222>  (1)..(1)
<223>  Orn

<220>
<221>  MISC_FEATURE
<222>  (2)..(2)
<223>  Xaa shows any amino acid.

<220>
<221>  MISC_FEATURE
<222>  (4)..(4)
<223>  Xaa shows any amino acid.

<400>  12
Xaa Xaa Arg Xaa
1


<210>  13
<211>  4
<212>  PRT
<213>  Artificial Sequence

<220>

<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (2)..(2)
<223>  Orn

<220>
<221>  MISC_FEATURE
<222>  (4)..(4)
<223>  Xaa shows any amino acid.

<400>  13
Xaa Xaa Arg Xaa
1


<210>  14
<211>  5
<212>  PRT
<213>  Artificial Sequence

<220>
<223>  Peptide having inhibitory action against AGE2 production

<220>
<221>  MISC_FEATURE
<222>  (1)..(1)
<223>  Orn

<220>
<221>  MISC_FEATURE
<222>  (3)..(3)
<223>  Xaa shows any amino acid.

<400>  14
Xaa Arg Xaa Lys Lys
1               5


<210>  15
<211>  5
<212>  PRT
<213>  Artificial Sequence

<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (3)..(3)
<223>  Xaa shows any amino acid.

<220>
<221>  MISC_FEATURE
<222>  (5)..(5)
<223>  Xaa shows any amino acid.

<400>  15
Xaa Arg Xaa Arg Xaa
1               5


<210>  16
<211>  5
<212>  PRT

<213>  Artificial Sequence

<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (4)..(4)
<223>  Xaa shows any amino acid.

<220>
<221>  MISC_FEATURE
<222>  (5)..(5)
<223>  Xaa shows any amino acid.

<400>  16
Xaa Lys Arg Xaa Xaa
1               5


<210>  17
<211>  5
<212>  PRT
<213>  Artificial Sequence

<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (5)..(5)
<223>  Xaa shows any amino acid.

<400>  17
Xaa Lys Lys Lys Xaa
1               5


<210>  18
<211>  5
<212>  PRT
<213>  Artificial Sequence

<220>
<223>  Peptide having inhibitory action against AGE2 production

<220>
<221>  MISC_FEATURE
<222>  (1)..(1)
<223>  Xaa shows any amino acid.

<220>
<221>  MISC_FEATURE
<222>  (2)..(2)
<223>  Orn

<220>
<221>  MISC_FEATURE
<222>  (3)..(3)
<223>  Xaa shows any amino acid.

<400>  18
Xaa Xaa Xaa Lys Lys
1               5

## Description

### Technical Field

The present invention relates to a novel agent for the prophylaxis or treatment of diabetic complications. More particularly, an agent for the prophylaxis or treatment of diabetic complications containing a certain kind of oligopeptide as an active ingredient, an AGE2 production inhibitor containing the oligopeptide, an AGE2 blood concentration reduction promoter containing the oligopeptide, and food containing the oligopeptide.

### Background Art

Diabetes is a metabolic abnormality caused by absolute or relative deficit of insulin, which is a sole hypoglycemic hormone, and is mainly characterized by sustained hyperglycemia. Persistence of hyperglycemic state not only further aggravates the metabolic abnormality due to insulin deficit, but also causes microangiopathy in kidney·nerve·retina and the like or macroangiopathy such as arteriosclerosis and the like, and markedly impairs the healthy life.

The basic treatment of diabetic patients is control of the blood glucose level. As the situation stands, however, no treatment with a hypoglycemic agent can provide a sufficient blood glucose management, and the prevalence of neuropathy, retinopathy, nephropathy and foot gangrene of the patients under diabetic treatments in Japan is as considerably high as 15.6%, 13.1%, 15.2% and 1.6%, respectively (Office For Life-Style Related Diseases Control, General Affairs Division, Health Service Bureau, Ministry of Health, Labor and Welfare, Heisei-14 diabetes survey, 2003). Thus, an attempt to prevent the onset of diabetic complications and suppress its progress is extremely important, besides the blood glucose management.

In recent years, involvement of advanced glycation endoproducts (AGEs) produced by a nonenzymatic glycation reaction in the onset of diabetic complications has been known (Stitt Exp Mol Pathol 75, 95-108, 2003). AGEs is a generic term for compounds produced by binding of protein or peptide in blood with glucose or a metabolite or autoxidation decomposition product thereof, and AGEs are known to directly induce cell dysfunction. Particularly, of AGEs, AGE2 produced from glyceraldehydes shows strong cytotoxicity, and is considered to be one of the substances important as promoting factors of the onset or progress of diabetic complications (Takeuchi et al., J Neuropathol Exp Neurol, 59, 1094-1105, 2000). As attempts to reduce cell dysfunction due to AGEs, 1) inhibition of AGEs production, 2) inhibition of formation of crosslinking structure of AGEs, 3) inhibition of interaction between AGEs and receptors thereof, and the like have been reported (Mene P et al., Am J Cardiovasc Drugs, 3, 315-20, 2003). As the situation stands, however, a sufficient therapeutic achievement has not been afforded as yet. In any case, since the starting point of cell dysfunction is the production of AGEs due to chronic hyperglycemia, the "inhibition of AGEs production" of 1) is considered to be one of the important approaches, next to the blood glucose control, as a means to prevent the onset of diabetic complications. However, an attempt focusing on the inhibition of production of AGE2 having particularly strong cytotoxicity has not been tried heretofore.

As an approach to alleviate cell dysfunction by inhibiting AGEs production, therapies using aminoguanidine, pyridoxamine, lysine (Jyothirmayi GN et al., Nephron, 87, 148-54, 2001), OPB-9195 (Kalousova M et al., Kidney Blood Press Res, 27, 18-28, 2004) and the like have been reported. However, they focus on the inhibition of production of carboxymethyllysine and glucose-derived AGE (AGE1), and clinically sufficient therapeutic achievements have not been made.

### Disclosure of the Invention

An attempt to prevent the onset and suppress the progress of diabetic complications in addition to the blood glucose management is extremely important. While some of the causes of diabetic complications are known, AGEs are considered to play an important role as causative substances deriving from hyperglycemia as mentioned above, and particularly, AGE2 having strong cytotoxicity is one of the strongly suspected causative substances. Accordingly, the problem to be solved by the present invention is provision of an agent for the prophylaxis or treatment of diabetic complications, which characteristically suppresses AGE2 production, or reduces blood concentration of AGE2.

The present inventors have conducted intensive studies in an attempt to solve the above-mentioned problem, and found that an oligopeptide comprising an amino acid having an amino group or a guadinino group in the side chain has AGE2 production inhibitory action and AGE2 blood concentration reduction-promoting action, which resulted in the completion of the present invention.

To be specific, the present inventors constructed various oligopeptides containing lysine, arginine and ornithine, which are amino acids having an amino group or a guadinino group in the side chain and studied the AGE2 production inhibitory action thereof. As a result, they have found that the oligopeptides containing such amino acids synergistically react with glyceraldehyde to form AGE2, when compared to these amino acids alone, and inhibit production of AGE2 from the coexisting normal blood protein and glyceraldehyde. The activity is far stronger than expected from the combination of lysine monomers, where the above-mentioned amino acids in the form of oligopeptide afford a synergistic effect. They have also found that administration to diabetes mouse model reduces AGE2 concentration of serum in a dose-dependent manner. It is considered that after these oligopeptides react with glyceraldehyde to give AGE2 in vivo, the AGE2 is easily eliminated or decomposed as compared to AGE2 made from a protein. Since oligopeptide to be used in the present invention suppresses the blood AGE2 level, it is effective for the alleviation or prophylaxis of diabetic complications.

Based on such findings, the present inventors have completed the present invention relating to a novel agent for the prophylaxis or treatment of diabetic complications.

Accordingly, the present invention relates to the following;
(1) an agent for the prophylaxis or treatment of diabetic complications, comprising, as an active ingredient, an oligopeptide containing at least two amino acid residues having an amino group or a guadinino group in the side chain;
(2) the agent of the above-mentioned (1), wherein the amino acid having an amino group or a guadinino group in the side chain is selected from the group consisting of lysine, arginine and ornithine;
(3) the agent of the above-mentioned (1) or (2), wherein the oligopeptide consists of 2 to 4 amino acid residues;
(4) the agent of any one of the above-mentioned (1) to (3), wherein the oligopeptide is in the form of a derivative;
(5) the agent of the above-mentioned (1), wherein the oligopeptide is a mixture of one or more kinds selected from the group consisting of Lys-Lys, Lys-Lys-Lys, Lys-Lys-Lys-Lys (SEQ ID NO: 1), and Arg-Lys;
(6) the agent of any one of the above-mentioned (1) to (5), which is used for at least one kind selected from the group consisting of diabetes, diabetic peripheral neuropathy, diabetic nephropathy, diabetic retinopathy, diabetic macroangiopathy and impaired glucose tolerance;
(7) an agent for inhibiting AGE2 production, which comprises, as an active ingredient, an oligopeptide containing at least two amino acid residues having an amino group or a guadinino group in the side chain;
(8) an agent for promoting reduction of AGE2 blood concentration, which comprises, as an active ingredient, an oligopeptide containing at least two amino acid residues having an amino group or a guadinino group in the side chain;
(9) a food for the prophylaxis or treatment of diabetic complications, inhibition of AGE2 production, and/or promotion of reduction of AGE2 blood concentration, which comprises, as an active ingredient, an oligopeptide containing at least two amino acid residues having an amino group or a guadinino group in the side chain;
(10) the food of the above-mentioned (9), having a label indicating its use for the prophylaxis or treatment of diabetic complications, inhibition of AGE2 production, and/or promotion of reduction of AGE2 blood concentration;
(11) a method for the prophylaxis or treatment of diabetic complications, which comprises administering, to a patient, an effective amount of an oligopeptide containing at least two amino acid residues having an amino group or a guadinino group in the side chain;
(12) a method of inhibiting AGE2 production, which comprises administering, to a patient, an effective amount of an oligopeptide containing at least two amino acid residues having an amino group or a guadinino group in the side chain;
(13) a method of promoting reduction of AGE2 blood concentration, which comprises administering, to a patient, an effective amount of an oligopeptide containing at least two amino acid residues having an amino group or a guadinino group in the side chain;
(14) use of an oligopeptide containing at least two amino acid residues having an amino group or a guadinino group in the side chain for the production of a drug for the prophylaxis or treatment of diabetic complications;
(15) use of an oligopeptide containing at least two amino acid residues having an amino group or a guadinino group in the side chain for the production of an agent for inhibiting AGE2 production;
(16) use of an oligopeptide containing at least two amino acid residues having an amino group or a guadinino group in the side chain for the production of an agent for promoting reduction of AGE2 blood concentration;
(17) a commercial package comprising a pharmaceutical composition comprising an oligopeptide containing at least two amino acid residues having an amino group or a guadinino group in the side chain and a pharmacologically acceptable carrier, and a written matter associated therewith, the written matter stating that the pharmaceutical composition can or should be used for the prophylaxis or treatment of diabetes;
(18) a commercial package comprising a pharmaceutical composition comprising an oligopeptide containing at least two amino acid residues having an amino group or a guadinino group in the side chain and a pharmacologically acceptable carrier, and a written matter associated therewith, the written matter stating that the pharmaceutical composition can or should be used for the inhibition of AGE2 production;
(19) a commercial package comprising a pharmaceutical composition comprising an oligopeptide containing at least two amino acid residues having an amino group or a guadinino group in the side chain and a pharmacologically acceptable carrier, and a written matter associated therewith, the written matter stating that the pharmaceutical composition can or should be used for the promotion of reduction of AGE2 blood concentration.

### Best Mode for Embodying the Invention

The agent for the prophylaxis or treatment of diabetic complications of the present invention contains, as an active ingredient, an oligopeptide containing at least two amino acid residues having an amino group or a guadinino group in the side chain, and may contain a pharmacologically acceptable carrier and the like as appropriate. In the present invention, as the amino acid having an amino group or a guadinino group in the side chain, lysine, arginine and ornithine can be mentioned, each of which may be a D-form, an L-form or a DL-form. From the aspect of easy availability, an L-form is preferable.

The oligopeptide to be used in the present invention is a peptide containing at least two amino acid residues having an amino group or a guadinino group in the side chain, which may be a peptide consisting only of an amino acid having an amino group or a guadinino group in the side chain or a peptide consisting of both an amino acid having an amino group or a guadinino group in the side chain and other amino acid. As the oligopeptide to be used in the present invention, a peptide preferably consisting of 2-5, more preferably 2-4, most preferably 2-3, amino acid residues can be mentioned.

Specifically, the oligopeptide to be used in the present invention contains at least 2 amino acid residues having an amino group or a guadinino group in the side chain, which is exemplified by, but not particularly limited to, a combination selected from Lys-Lys, Lys-Arg, Orn-Lys, Orn-Arg, Lys-Lys-Lys, X-Lys-Lys, Lys-X-Lys, Lys-X-Arg, Lys-X-Orn, Arg-X-Orn, Lys-Lys-Lys-Lys (SEQ ID NO: 1), Lys-X-Lys-X (SEQ ID NO: 2), X-Lys-Lys-X (SEQ ID NO: 3), Arg-X-Arg-X (SEQ ID NO: 4), X-Arg-Arg-X (SEQ ID NO: 5), X-Lys-Arg-X (SEQ ID NO: 6), Lys-X-Arg-X (SEQ ID NO: 7), X-Lys-X-Arg (SEQ ID NO: 8), X-Orn-Lys-X (SEQ ID NO: 9), Orn-X-Lys-X (SEQ ID NO: 10), X-Orn-X-Lys (SEQ ID NO: 11), Orn-X-Arg-X (SEQ ID NO: 12), X-Orn-Arg-X (SEQ ID NO: 13), Orn-Arg-X-Lys-Lys (SEQ ID NO: 14), X-Arg-X-Arg-X (SEQ ID NO: 15), X-Lys-Arg-X-X (SEQ ID NO: 16), X-Lys-Lys-Lys-X (SEQ ID NO: 17), X-Orn-X-Lys-Lys (SEQ ID NO: 18) (X is an amino acid selected from all amino acids and the above-mentioned sequences include both orders of from the N-terminal to the C-terminal, and from the C-terminal to the N-terminal) and the like. Examples of preferable oligopeptide include an oligopeptide selected from the group consisting of Lys-Lys, Lys-Lys-Lys, Lys-Lys-Lys-Lys (SEQ ID NO: 1), and Arg-Lys (the above-mentioned sequences consist of L-form amino acids in the order from the N-terminal to the C-terminal). In addition, one or more kinds of oligopeptides may be used in a mixture.

The oligopeptide to be used in the present invention can be obtained by appropriately using a known technique such as (1) a chemical synthesis method, (2) a synthesis method by enzymatic reaction, and the like. Since the oligopeptide to be used in the present invention contains a relatively small number of amino acid residues of 2 to 5, a chemical synthesis method is convenient.

In the case of chemical synthesis, the oligopeptide is synthesized or semisynthesized using a peptide synthesizer. As the chemical synthesis method, for example, solid-phase peptide synthesis can be mentioned. The oligopeptide synthesized in such manner can be purified by a conventional method, such as ion exchange chromatography, reversed-phase high performance liquid chromatography, affinity chromatography and the like. Such solid-phase peptide synthesis and the subsequent peptide purification are well known in this technical field.

When the oligopeptide to be used in the present invention is dipeptide or tripeptide, the oligopeptide can also be produced by an enzymatic reaction. For example, the method described in WO 2004/011653 can be used. Namely, an amino acid or dipeptide wherein one amino acid or carboxyl-terminal of dipeptide is esterified or amidated is reacted with an amino acid wherein an amino group is free (e.g., amino acid wherein carboxyl group is protected) in the presence of a peptide-producing enzyme, and the produced dipeptide or tripeptide is purified. As the peptide producing enzyme, a culture of a microorganism having an ability to produce peptide, a microorganism fungus body separated from the culture, a treated fungus body of the microorganism and a peptide-producing enzyme derived from the microorganism can be mentioned.

When the oligopeptide to be used in the present invention is contained in a food, an oligopeptide produced by the above-mentioned method may be added to the food, or may be added as a protein decomposition product. For example, a protein containing a large amount of lysine and arginine as constituent amino acids may be hydrolyzed with an acid or protease to give the above-mentioned oligopeptide, which may be directly added to the food.

The oligopeptide to be used in the present invention also includes oligopeptide derivatives. Specifically, it may be in the form of a prodrug obtained by appropriate modification to suppress decomposition during absorption when oligopeptide is orally administered, which includes one wherein a terminal amino group such as N-terminal of peptide, amino group in the side chain and the like is protected with a protecting group such as acyl group, alkyl group and the like, one wherein C-terminal carboxylic acid of peptide is protected with a protecting group such as ester group and the like, one wherein a part of amino acids in the oligopeptide is substituted by the corresponding D-amino acid, and the like. As an example of acylation, addition of a linear or branched acyl group having 6 to 10 carbon atoms to a lysine residue side chain can be mentioned. The derivatives of these further include one wherein the carboxyl group of the C-terminal amino acid residue is substituted by an alcohol group, and one wherein the C-terminal amino acid residue is substituted by an amino group. Conversion to a derivative form in this way is sometimes preferable, since it may improve the in vivo kinetics. As a derivative, lysine also includes hydroxylysine.

For the formation and removal of the protecting group, a known method can be applied. As a representative amino protecting group, for example, an acyl group (formyl group, acetyl group and the like), an alkyl group (isopropyl group and the like), an alkoxycarbonyl group (butoxycarbonyl group and the like), a fluorenylmethoxycarbonyl group, a benzyloxycarbonyl group and the like can be mentioned, where the amino group in the N-terminal amino acid residue and basic amino acid residue side chain is preferably protected. As a representative carboxyl protecting group, for example, benzyl ester, methyl ester, t-butyl ester, p-nitrophenyl ester and the like can be mentioned, where the carboxyl group in the C-terminal amino acid residue and acidic amino acid residue side chain is preferably protected.

The oligopeptide to be used in the present invention also encompasses a salt. When the oligopeptide of the present invention can form a salt, the salt only needs to be pharmacologically acceptable and, for example, when an acidic group, such as a carboxyl group and the like, is present, ammonium salt, salts with alkali metal such as sodium, potassium and the like, salts with alkaline earth metal such as calcium, magnesium and the like, aluminum salts, zinc salts, salts with organic amine such as triethylamine, ethanolamine, morpholine, pyrrolidine, piperidine, piperazine, dicyclohexylamine and the like, and salts with basic amino acid such as arginine, lysine and the like can be mentioned. When a basic group is present, salts with inorganic acid such as hydrochloric acid, sulfuric acid, phosphoric acid, nitric acid, hydrogen bromide acid and the like, salts with organic carboxylic acid such as acetic acid, citric acid, benzoic acid, maleic acid, fumaric acid, tartaric acid, succinic acid, tannic acid, butyric acid, hibenzoic acid, pamoic acid, enanthic acid, decanoic acid, teoclic acid, salicylic acid, lactic acid, oxalic acid, mandelic acid, malic acid and the like, and salts with organic sulfonic acid such as methanesulfonic acid, benzenesulfonic acid, p-toluenesulfonic acid and the like can be mentioned.

The oligopeptide to be used in the present invention inhibits AGE2 production both in vitro and *in vivo* (in blood), and promotes reduction of AGE2 blood concentration in vivo. While the above-mentioned oligopeptide itself can be reacted to become AGE2, it is assumed that the oligopeptide converted to AGE2 inhibits production of AGE2 from a protein or peptide having a greater molecular weight. While the above-mentioned oligopeptide inhibits production of AGE2 from blood protein or peptide in vivo (in blood) and itself becomes AGE2, which is considered to be quickly discharged from the body or decomposed to promote reduction of AGE2 concentration in blood.

The oligopeptide to be used in the present invention has superior AGE2 production inhibitory action and AGE2 blood concentration reduction-promoting action in mammals such as bovine, horse, swine, dog, cat, mouse, rat and the like, including human, and therefore, is useful for the prophylaxis or treatment of a disease for which the inhibition of AGE2 production and/or promotion of blood concentration reduction are/is effective, specifically diabetic complications.

As the target diseases of the agent for the prophylaxis or treatment of diabetic complications of the present invention, diabetes, diabetic peripheral neuropathy, diabetic nephropathy, diabetic retinopathy, diabetic macroangiopathy, impaired glucose tolerance and the like can be mentioned, though not limited thereto.

The administration method of the agent for the prophylaxis or treatment of diabetic complications of the present invention may be oral or parenteral, and as the dosage form, oral preparations such as tablet, powder, pill, granule, sugar coated agent, emulsifier, capsule, syrup, liquid, suspension and the like, and parenteral agents such as injection, aerosol, suppository and the like can be mentioned, with preference given to oral dosage form from the aspect of convenience.

The pharmaceutical preparation of the present invention can contain the above-mentioned oligopeptide and a pharmacologically acceptable carrier. As the pharmacologically acceptable carrier, for example, excipients (e.g., lactose, sucrose, starch, D-mannitol etc.), binders (e.g., cellulose, sucrose, dextrin, hydroxypropylcellulose, polyvinylpyrrolidone etc.), disintegrants (e.g., starch, carboxymethylcellulose etc.), lubricants (e.g., magnesium stearate etc.), surfactants (e.g., sodium lauryl sulfate etc.), solvents (e.g., water, brine, soy bean oil etc.), ointment bases (e.g., paraffin etc.), isotonizing agents (e.g., sodium chloride etc.), preservatives (e.g., p-hydroxybenzoic acid ester etc.) and the like known to those of ordinary skill in the art can be mentioned, though not limited thereto.

In the case of an injection, from the viewpoint of stability, it can be filled in a vial and the like and then frozen, the moisture is removed by a conventional freeze-dry treatment, and a liquid can be prepared again from the lyophilizate immediately before use.

While the dose of the agent for the prophylaxis or treatment of diabetic complications of the present invention varies depending on the age, body weight and severity of the disease of patients, for oral administration, the above-mentioned oligopeptide is generally appropriately administered to an adult in an amount of 1 mg - 50 g, preferably 10 mg - 20 g, particularly preferably 50 mg - 15 g, in one to several portions for one day. One kind or several kinds of the above-mentioned oligopeptides may be contained in a preparation. When several kinds are used, the total amount only needs to fall within the above-mentioned range.

The present invention also relates to a food (including beverage) containing the above-mentioned oligopeptide. Since the present invention contains amino acid as an active ingredient, it is superior in safety and can be used conveniently in the form of food. Such food includes those taken as food and health food, food additive, supplement etc. taken for the purpose of supplementing nutrition. Use of the present invention for food is not particularly difficult and, for example, can be eaten or drunk after mixing with juice, milk, confectionery, jelly and the like. For use as health food, food additive, supplement etc., for example, it can be prepared into the form of tablet, capsule, powder, granule, suspension, chewable agent, syrup and the like. Furthermore, it is possible to provide such food as food with health claims, particularly specified health food and the like, where the food with health claims includes one under a label indicating use for the prophylaxis or treatment of diabetic complications, inhibition of AGE2 production, and/or promotion of reduction of AGE2 blood concentration.

One or more kinds of the above-mentioned oligopeptides may be added to the food of the present invention, or may be added as a decomposition product (hydrolysis product) of protein (when the above-mentioned oligopeptide is contained). The food of the present invention is desirably taken by about 10 mg - 50. g per day, particularly preferably about 50 mg - 15 g, based on the above-mentioned oligopeptide. When several kinds are used, the total amount only needs to be within the above-mentioned range.

The agent for the prophylaxis or treatment of diabetic complications of the present invention can also be used in combination with a conventionally-used therapeutic agent for diabetes or agent for the prophylaxis or treatment of diabetic complications. As the conventionally-used therapeutic agent for diabetes, and the agent for the prophylaxis or treatment of diabetic complications, for example, a combination of one or more kinds of and mixtures of insulin preparations, insulin derivatives, insulin-like action agents, insulin secretagogues, insulin sensitizers, biguanides, gluconeogenesis inhibitors, sugar absorption inhibitors, renal sugar reabsorption inhibitors, β3 adrenoceptor agonists, glucagon-like peptide-1 (7-37), glucagon-like peptide-1 (7-37) analogs, glucagon-like peptide-1 receptor agonists, dipeptidylpeptidase IV inhibitors, aldose reductase inhibitors, advanced glycation end product-production inhibitors, glycogen synthase kinase-3 inhibitors, glycogen phosphorylase inhibitors, hypolipidemic agents, anorexigenic agents, lipase inhibitors, antihypertensive agents, peripheral circulation improving agents, antioxidants, therapeutic drugs for diabetic neuropathy and the like can be mentioned.

Examples of concrete pharmaceutical compounds to be used in combination and the suitable diseases to be treated are given in the following. However, the subject matter of the present invention is not limited to them, and the concrete compounds include a free form and/or other pharmacologically acceptable salt thereof.

As the insulin preparations, NPH, lente, ultralente, lung absorbable insulin and the like can be mentioned.

The insulin derivative is a protein or peptide derived from insulin, which maintains the insulin action, such as lispro, B10Asp, glargine and the like.

The insulin-like action agent refers to those other than insulin derivatives, which exhibit blood glucose lowering action by exhibiting the physiological action of insulin to a certain degree, such as intracellular sugar uptake promoting action and the like, without depending on insulin and, for example, insulin receptor kinase stimulating drugs (e.g., L-783281, TER-17411, CLX-0901, KRX-613 and the like), vanadium and the like can be mentioned.

The insulin secretagogue refers to those that exhibit blood glucose lowering action by acting on the pancreatic β cell, and increasing the insulin secretion in blood and, for example, sulfonylurea (e.g., tolbutamide, chlorpropamide, tolazamide, acetohexamide, gliclazide, glimepiride, glipizide, glibenclamide (glyburide) and the like), meglitinides (e.g., nateglinide, repaglinide, mitiglinide and the like), ATP-sensitive potassium channel blockers other than sulfonylurea-meglitinides (e.g., BTS-67-582 and the like) and the like can be mentioned.

The insulin sensitizer refers to those that exhibit blood glucose lowering action by enhancing the action of insulin in the insulin target tissues and, for example, peroxisome proliferator-activated receptor (PPAR)γ agonists (e.g., thiazolidinedione compounds such as pioglitazone, rosiglitazone, troglitazone, siglitazone and the like, non-thiazolidinedione compounds such as GI-262570, GW-1929, JTT-501, YM-440 and the like), PPARγ antagonists (e.g., bisphenol A diglycidyl ether, LG-100641 and the like), PPARα agonists (fibrate compounds such as clofibrate, bezafibrate, clinofibrate and the like, or non-fibrate compounds and the like), PPARα/γ agonists (e.g., KRP-297 and the like), retinoid X receptor agonists (e.g., LG-100268 and the like), retinoid X receptor antagonists (e.g., HX531 and the like), protein tyrosine phosphatase-1B inhibitors (e.g., PTP-112 and the like) and the like can be mentioned.

The biguanide refers to those that exhibit blood glucose lowering action by gluconeogenesis suppressing action in the liver, anaerobic glycolysis promoting action in tissues or insulin resistant activity in the periphery and the like and, for example, metformin, phenformin, buformin and the like can be mentioned.

The gluconeogenesis inhibitor refers to those that exhibit blood glucose lowering action by mainly inhibiting gluconeogenesis and, for example, glucagon secretion suppressors (e.g., M&B 39890A and the like), glucagon receptor antagonists (e.g., CP-99711, NNC-92-1687, L-168049, BAY27-9955 and the like), glucose-6-phosphatase inhibitors and the like can be mentioned.

The sugar absorption inhibitor refers to those that exhibit blood glucose lowering action by inhibiting enzyme digestion of carbohydrate contained in food in the gastrointestinal tract, and inhibiting or delaying absorption of sugar into the body and, for example, α-glucosidase inhibitors (e.g., acarbose, voglibose, miglitol and the like), α-amylase inhibitors (e.g., AZM-127 and the like) and the like can be mentioned.

The renal sugar reabsorption inhibitor refers to those that exhibit blood glucose lowering action by inhibiting reabsorption of sugar in the renal tubule and, for example, sodium-dependent glucose transporter inhibitors (e.g., T-1095, phloridzin and the like) and the like can be mentioned.

The β3 adrenoceptor agonist refers to those that exhibit obesity or hyperinsulinemia-improving effect by stimulating β3 adrenoceptor in adipocytes, promoting fatty acid oxidation and causing energy consumption and, for example, CL-316243, TAK-677 and the like can be mentioned.

As the glucagon-like peptide-1 (7-37) analog, for example, exendin-4, NN-2211 and the like can be mentioned; as the glucagon-like peptide-1 receptor agonist, for example, AZM-134 and the like can be mentioned; and as the dipeptidylpeptidase IV inhibitor, for example, NVP-DPP-728 and the like can be mentioned. The glucagon-like peptide-1 analog, glucagon-like peptide-1 receptor agonist, dipeptidylpeptidase IV inhibitor and glucagon-like peptide-1 refer to those that exhibit diabetes improving effect by mimicking or enhancing the action of glucagon-like peptide-1 in the cell.

The aldose reductase inhibitor refers to one that, from such inhibitors preferable for the treatment of diabetic complications, decreases cellular sorbitol accumulated in excess due to facilitated polyol metabolic pathway resulting from the retention of the hyperglycemic state, which is observed in a tissue where diabetic complications are developed, by inhibiting aldose reductase and, for example, epalrestat, tolrestat, fidarestat, zenerestat and the like can be mentioned.

The advanced glycation end product-production inhibitor refers to one that, from such inhibitors preferable for the treatment of diabetic complications, alleviates cell dysfunction by inhibiting the production of advanced glycation end products facilitated by the retention of hyperglycemic state in the diabetic state, and the agent for the prophylaxis or treatment of diabetic complications of the present invention is also encompassed in this category. For a combined use with the agent for the prophylaxis or treatment of diabetic complications of the present invention, the inhibitor is other than the agent for the prophylaxis or treatment of diabetic complications of the present invention and, for example, NNC-39-0028, OPB-9195 and the like can be mentioned.

As the glycogen synthase kinase-3 inhibitor, for example, SB-216763, CHIR-98014 and the like can be mentioned, and as the glycogen phosphorylase inhibitor, for example, CP-91149 and the like can be mentioned.

As the hypolipidemic agent, for example, hydroxymethylglutaryl-coenzyme A reductase inhibitors (e.g., pravastatin, simvastatin, fluvastatin, atorvastatin and the like), fibrates (e.g., clofibrate, bezafibrate, simfibrate and the like), bile acid-binding resins and the like can be mentioned.

As the anorexigenic drug, for example, sibutramine, mazindol and the like can be mentioned, and as the lipase inhibitor, for example, orlistat and the like can be mentioned.

As the antihypertensive agent, for example, angiotensin converting enzyme inhibitors (e.g., captopril, alacepril and the like), angiotensin II receptor antagonists (e.g., candesartan cilexetil, valsartan and the like), calcium antagonists (e.g., cilnidipine, amlodipine, nicardipine and the like), diuretics (e.g., trichlormethiazide, spironolactone and the like), sympatholytic agents (e.g., clonidine, reserpine and the like) and the like can be mentioned.

As the peripheral circulation improvement agent, for example, ethyl icosapentate and the like can be mentioned.

As the antioxidant, for example, lipoic acid, probucol and the like can be mentioned. .

As the therapeutic drug for diabetic neuropathy, for example, mecobalamin, mexiletine hydrochloride and the like can be mentioned.

### Examples

The present invention is explained in more detail in the following by referring to Examples, which are given only for the explanation of the present invention and not to be construed as limitative. In the chemical formula of the oligopeptide used in the Examples, amino acid was an L-form, and the order of sequence was from the N-terminal to the C-terminal.

### [Example 1] AGE2-producing capability by reaction of oligopeptide with glyceraldehyde

To 0.1M phosphate buffer (pH 7.4) containing 10 mM of any of Lys, Lys-Lys, Lys-Lys-Lys and Lys-Lys-Lys-Lys (SEQ ID NO: 1) was added 100 mM glyceraldehyde and the mixture was reacted at 37°C for 1 week. One week later, the AGE2 concentration of the reaction mixture was measured. The measurement was performed by the ELISA method using an anti-AGE2 polyclonal antibody prepared from rabbit, according to a conventional method.

### (Results)

**(Table 1)**

| Amino acid or peptide | Lys | Lys-Lys | Lys-Lys-Lys | Lys-Lys-Lys-Lys |
|---|---|---|---|---|
| AGE2 concentration (U/ml) | 0.47 | 4.7 | 6.8 | 14.2 |

It has been clarified that the AGE2-producing capacity of each peptide is enhanced as the number of Lys increases. The production capacity in Lys-Lys was about 10 times that of Lys alone, where the AGE2 production amount increased synergistically as the number increased. Therefore, it was confirmed that an oligopeptide of amino acid having an amino group in the side chain synergistically expressed AGE2-production promoting action.

### [Example 2] Capability of oligopeptide to inhibit AGE2 production by glyceraldehyde and serum albumin

To phosphate buffer (pH 7.4) containing 10 mM of glyceraldehyde and 10 mg/ml of bovine serum albumin was added any of Lys, Lys-Lys, Lys-Lys-Lys, Lys-Lys-Lys-Lys (SEQ ID NO: 1), Arg and Arg-Lys to 0, 0.1, 0.3, 1.0 or 3.0 mM and the mixture was reacted at 37°C for 3 days. Three days later, AGE2 concentration of the reaction mixture was measured, and IC50 value of inhibition of AGE2 production by each peptide or amino acid was calculated. The AGE2 measurement was performed by the ELISA method using an anti-AGE2 polyclonal antibody prepared from rabbit, according to a conventional method.

### (Results)

**(Table 2)**

| Amino acid or peptide | Lys | Lys-Lys | Lys-Lys- Lys | Lys-Lys- Lys-Lys | Arg | Arg-Lys |
|---|---|---|---|---|---|---|
| IC50 (mM) | 2.2 | 0.56 | 0.53 | 0.62 | 1.9 | 0.78 |

It has been clarified that AGE2 obtained by mixing and reacting glyceraldehyde with serum albumin for a given length of time inhibits accumulation of albumin converted to AGE2, due to the coexistence of Lys or Arg and oligopeptide containing same. Therefore, it was confirmed that the activity of oligopeptide synergistically increased as compared to Lys alone.

### [Example 3] Serum AGE2 concentration reduction action by Lys-Lys in diabetic mouse model (db/db mouse)

7-Week-old male db/db mice were randomly divided into 4 groups (3 per group), distilled water, 10 mM Lys-Lys solution, 30 mM.Lys-Lys solution, or 100 mM Lys-Lys solution was freely given for 10 days as drinking water, blood samples were collected from the heart, and the serum AGE2 concentration was measured. The measurement was performed by the ELISA method using an anti-AGE2 polyclonal antibody prepared from rabbit, according to a conventional method.

### (Results)

**(Table 3)**

| administration concentration | 0 mM | 10 mM | 30 mM | 100 mM |
|---|---|---|---|---|
| serum AGE2 concentration (U/ml) | 4.0±0.5 | 3.5±0.6 | 2.3±0.4 | 1.7±0.2 |

The serum AGE2 concentration decreased in a manner dependent on the Lys-Lys concentration of the Lys-Lys solution taken.

Therefore, judging from the results including those of the above-mentioned Examples 1 and 2, it is considered that Lys-Lys itself was converted to AGE2, and discharged from the body without accumulation, or decomposed to cause reduction of the serum AGE2 concentration.

### Industrial Applicability

The novel agent for the prophylaxis or treatment of diabetic complications of the present invention has superior AGE2 production inhibitory action and blood AGE2 concentration reduction promoting action and is useful for the prophylaxis and/or treatment of diabetic complications. According to the present invention, moreover, a superior AGE2 production inhibitor and a blood AGE2 concentration reduction promoter can be provided. According to the present invention, furthermore, a food for the prophylaxis or treatment of diabetic complications, inhibition of AGE2 production, and/or promotion of reduction of AGE2 blood concentration can be provided.

Since the present invention can provide a novel drug for the prophylaxis or treatment of diabetic complications, it can be used in the pharmaceutical field.

### Sequence Listing Free Text

SEQ ID NO: 1: A peptide having AGE2 production inhibitory action.
SEQ ID NO: 2: A peptide having AGE2 production inhibitory action, wherein Xaa is any amino acid.
SEQ ID NO: 3: A peptide having AGE2 production inhibitory action, wherein Xaa is any amino acid.
SEQ ID NO: 4: A peptide having AGE2 production inhibitory action, wherein Xaa is any amino acid.
SEQ ID NO: 5: A peptide having AGE2 production inhibitory action, wherein Xaa is any amino acid.
SEQ ID NO: 6: A peptide having AGE2 production inhibitory action, wherein Xaa is any amino acid.
SEQ ID NO: 7: A peptide having AGE2 production inhibitory action, wherein Xaa is any amino acid.
SEQ ID NO: 8: A peptide having AGE2 production inhibitory action, wherein Xaa is any amino acid.
SEQ ID NO: 9: A peptide having AGE2 production inhibitory action, wherein the first Xaa is any amino acid, the second Xaa is ornithine, and the 4th Xaa is any amino acid.
SEQ ID NO: 10: A peptide having AGE2 production inhibitory action, wherein the first Xaa is ornithine, the second Xaa is any amino acid, and the 4th Xaa is any amino acid.
SEQ ID NO: 11: A peptide having AGE2 production inhibitory action, wherein the first Xaa is any amino acid, the second Xaa is ornithine, and the 3rd Xaa is any amino acid.
SEQ ID NO: 12: A peptide having AGE2 production inhibitory action, wherein the first Xaa is ornithine, the second Xaa is any amino acid, and the 4th Xaa is any amino acid.
SEQ ID NO: 13: A peptide having AGE2 production inhibitory action, wherein the first Xaa is any amino acid, the second Xaa is ornithine, and the 4th Xaa is any amino acid.
SEQ ID NO: 14: A peptide having AGE2 production inhibitory action, wherein the first Xaa is ornithine, and the 3rd Xaa is any amino acid.
SEQ ID NO: 15: A peptide having AGE2 production inhibitory action, wherein Xaa is any amino acid.
SEQ ID NO: 16: A peptide having AGE2 production inhibitory action, wherein Xaa is any amino acid.
SEQ ID NO: 17: A peptide having AGE2 production inhibitory action, wherein Xaa is any amino acid.
SEQ ID NO: 18: A peptide having AGE2 production inhibitory action, wherein the first Xaa is any amino acid, the second Xaa is ornithine, and the 3rd Xaa is any amino acid.

This application is based on a patent application No. 2004-091767 filed in Japan (filing date: March 26, 2004), the contents of which are incorporated in full herein by this reference.

## Claims

1. An agent for the prophylaxis or treatment of diabetic complications, comprising, as an active ingredient, an oligopeptide containing at least two amino acid residues having an amino group or a guadinino group in the side chain.

2. The agent of claim 1, wherein the amino acid having an amino group or a guadinino group in the side chain is selected from the group consisting of lysine, arginine and ornithine.

3. The agent of claim 1 or 2, wherein the oligopeptide consists of 2 to 4 amino acid residues.

4. The agent of any one of claims 1 to 3, wherein the oligopeptide is in the form of a derivative.

5. The agent of claim 1, wherein the oligopeptide is a mixture of one or more kinds selected from the group consisting of Lys-Lys, Lys-Lys-Lys, Lys-Lys-Lys-Lys (SEQ ID NO: 1), and Arg-Lys.

6. The agent of any one of claims 1 to 5, which is used for at least one kind selected from the group consisting of diabetes, diabetic peripheral neuropathy, diabetic nephropathy, diabetic retinopathy, diabetic macroangiopathy and impaired glucose tolerance.

7. An agent for inhibiting AGE2 production, which comprises, as an active ingredient, an oligopeptide containing at least two amino acid residues having an amino group or a guadinino group in the side chain.

8. An agent for promoting reduction of AGE2 blood concentration, which comprises, as an active ingredient, an oligopeptide containing at least two amino acid residues having an amino group or a guadinino group in the side chain.

9. A food for the prophylaxis or treatment of diabetic complications, inhibition of AGE2 production, and/or promotion of reduction of AGE2 blood concentration, which comprises, as an active ingredient, an oligopeptide containing at least two amino acid residues having an amino group or a guadinino group in the side chain.

10. The food of claim 9, having a label indicating its use for the prophylaxis or treatment of diabetic complications, inhibition of AGE2 production, and/or,promotion of reduction of AGE2 blood concentration.

11. A method for the prophylaxis or treatment of diabetic complications, which comprises administering, to a patient, an effective amount of an oligopeptide containing at least two amino acid residues having an amino group or a guadinino group in the side chain.

12. A method of inhibiting AGE2 production, which comprises administering, to a patient, an effective amount of an oligopeptide containing at least two amino acid residues having an amino group or a guadinino group in the side chain.

13. A method of promoting reduction of AGE2 blood concentration, which comprises administering, to a patient, an effective amount of an oligopeptide containing at least two amino acid residues having an amino group or a guadinino group in the side chain.

14. Use of an oligopeptide containing at least two amino acid residues having an amino group or a guadinino group in the side chain for the production of a drug for the prophylaxis or treatment of diabetic complications.

15. Use of an oligopeptide containing at least two amino acid residues having an amino group or a guadinino group in the side chain for the production of an agent for inhibiting AGE2 production.

16. Use of an oligopeptide containing at least two amino acid residues having an amino group or a guadinino group in the side chain for the production of an agent for promoting reduction of AGE2 blood concentration.

17. A commercial package comprising a pharmaceutical composition comprising an oligopeptide containing at least two amino acid residues having an amino group or a guadinino group in the side chain and a pharmacologically acceptable carrier, and a written matter associated therewith, the written matter stating that the pharmaceutical composition can or should be used for the prophylaxis or treatment of diabetes.

18. A commercial package comprising a pharmaceutical composition comprising an oligopeptide containing at least two amino acid residues having an amino group or a guadinino group in the side chain and a pharmacologically acceptable carrier, and a written matter associated therewith, the written matter stating that the pharmaceutical composition can or should be used for the inhibition of AGE2 production.

19. A commercial package comprising a pharmaceutical composition comprising an oligopeptide containing at least two amino acid residues having an amino group or a guadinino group in the side chain and a pharmacologically acceptable carrier, and a written matter associated therewith, the written matter stating that the pharmaceutical composition can or should be used for the promotion of reduction of AGE2 blood concentration.
